# EUROPEAN PATENT APPLICATION

(11) **EP 2 343 360 A1**
(43) Date of publication of application: **13.07.2011**
(21) Application number: 09816124.3
(22) Date of filing: 18.09.2009
(51) Int. Cl.: C12M 1/00, B04B 5/02, B04B 11/04, B04B 15/12, C12M 1/10

(54) **CENTRIFUGE CONTAINER**

(30) Priority: 24.09.2008 JP 2008244403
(71) Applicant: Olympus Corporation, Shibuya-ku Tokyo 151-0072 (JP); Cytori Therapeutics, Inc., San Diego, California 92121 (US)
(72) Inventor: KINOSHITA, Aki, Tokyo 151-0072 (JP); ARM, Douglas M., Carlsbad, California 92009 (US); SHANAHAN, Robert K., Carlsbad, California 92009 (US); FORNACE, Lucas V., La Jolla, California 92037 (US)
(74) Representative: Gunzelmann, Rainer
(86) International application number: PCT/JP2009/066371
(87) International publication number: WO 2010/035709

(57) **Abstract**

A cell suspension that contains a cell group from which unwanted components have been removed by efficiently washing the cell group is recovered. A centrifuge container (1) includes a cylindrical container main body (2) that accommodates a cell suspension and is rotated with a bottom portion (2a) being directed radially outward; a supernatant suction tube (4) that has a first opening (4a) at a position in the depth direction of the container main body (2) and that suctions a supernatant obtained by centrifuging the cell suspension, in the radial direction of the container main body (2); and a washing-fluid discharge tube (3) and the supernatant suction tube (4) that have a second opening (3a) at a position in the depth direction of the container main body (2) and that discharge a washing fluid in the axial direction toward the bottom portion (2a) of the container main body (2).

## Description

### {Technical Field}

The present invention relates to a centrifuge container.

### {Background Art}

There is a conventionally known centrifuge separator in which a centrifuge container accommodating a cell suspension in which fat-derived cells are isolated by breaking down fat tissues is rotated around an axis located away from the centrifuge container, thereby separating components contained in the cell suspension according to their specific gravities (see PTL 1).
The centrifuge container is formed into a substantially cylindrical shape one end of which is closed. When the centrifuge container is rotated with the closed end being directed radially outward, components with higher specific gravities are moved to the closed end and are separated in descending order of specific gravity from the closed end.

### {Citation List}

### {Patent Literature}

{PTL 1} PCT International Publication No. WO 05/012480 Pamphlet

### {Summary of Invention}

### {Technical Problem}

One problem, however, is that a cell group separated in a bottom portion of the centrifuge container when the cell suspension is centrifuged is formed into a centrifugally solidified pellet. Specifically, if the cell group is formed into a pellet, it is difficult to remove the centrifuged cell group from the centrifuge container by suction. Furthermore, there is a case where the cell group is formed into a pellet while unwanted components, such as proteolytic enzyme and fat, are incorporated in the cell group during the centrifugation, and, in that case, it is difficult to remove the unwanted components.

The present invention has been made in view of the above-described circumstances, and an object thereof is to provide a centrifuge container capable of recovering a cell suspension that contains a cell group from which unwanted components have been removed by efficiently washing the cell group.

### {Solution to Problem}

In order to achieve the above-described object, the present invention provides the following solutions.
According to one aspect, the present invention provides a centrifuge container including: a cylindrical container main body that accommodates a cell suspension and is rotated with a bottom portion being directed radially outward; a supernatant suction tube that has a first opening at a position in the depth direction of the container main body and that suctions a supernatant obtained by centrifuging the cell suspension, from the first opening, in the radial direction of the container main body; and a washing-fluid discharge tube that has a second opening at a position in the depth direction of the container main body and that discharges a washing fluid from the second opening, in the axial direction toward the bottom portion of the container main body.

According to the aspect of the present invention, when a cell suspension is accommodated in the container main body and the container main body is rotated with the bottom portion being directed radially outward, components contained in the cell suspension are separated according to their specific gravities. In this case, a cell group with a higher specific gravity is collected at the lowermost layer in the bottom portion of the container main body, and a supernatant, such as blood plasma (also including a culture medium, saline, and a cell isotonic fluid), is collected at an upper layer. The cell group is centrifugally solidified to form a pellet.

In this state, the container main body is disposed along the vertical direction such that the bottom portion is directed to the lower side, and the supernatant is suctioned through the supernatant suction tube. Since the supernatant suction tube suctions the supernatant in the radial direction from the first opening, it is possible to discharge only the supernatant to the outside of the container main body, without suctioning the cell group collected in the bottom portion of the container main body.

Next, a washing fluid is supplied to the container main body through the washing-fluid discharge tube. Since the washing-fluid discharge tube discharges the washing fluid in the axial direction toward the bottom portion of the container main body, the washing fluid is blown out to the pellet-like cell group solidified in the bottom portion of the container main body, thus unsolidifying and spreading the pellet-like cell group. Thus, the cell group in which unwanted components have been incorporated at the time of centrifugation is unsolidified, and the unwanted components can be released in the washing fluid. Then, centrifugation is executed again, thereby reducing the concentration of the unwanted components incorporated in the cell group.

In the above-described aspect, the supernatant suction tube and the washing-fluid discharge tube may be provided with a common path through which the supernatant and the washing fluid flow, and a valve that is provided at the end of the common path, that opens the second opening when the washing fluid is discharged, and that closes the second opening when the supernatant is suctioned.

By doing so, in order to discharge the washing fluid to the container main body after centrifugation, the valve provided at the end of the common path is opened, and the washing fluid is discharged in the axial direction toward the bottom portion of the container main body and is blown out to the pellet-like cell group. Then, the cell group is unsolidified, and the unwanted components are discharged. In order to discharge the supernatant after the centrifugation, suction is performed through the common path, thus closing the valve and suctioning the supernatant in the radial direction. Therefore, it is possible to discharge the supernatant without avoiding suction of the cell group separated in the bottom portion. When the supernatant suction tube and the washing-fluid discharge tube are integrally formed, it is possible to simplify the structure by reducing the number of component parts.

Furthermore, the above-described aspect may further include a cell suction tube that has a third opening close to the bottom portion of the container main body and that suctions, from the third opening, a cell suspension that contains a cell group centrifuged in the bottom portion.
By doing so, it is possible to suction the cell suspension containing the cell group eventually centrifuged in the bottom portion of the container main body, from the third opening through the cell suction tube, to recover it to the outside of the container main body.

Furthermore, the above-described aspect may further include a suspension supply tube for supplying, to the container main body, a cell suspension that contains a cell group to be centrifuged.
By doing so, it is possible to introduce the cell suspension to the container main body through the suspension supply tube, and to perform processing from the introduction of the cell suspension to the separation of the cell group under an aseptic condition, without opening the lid of the container main body.

Furthermore, in the above-described structure, the suspension supply tube may be formed of piping common to the washing-fluid discharge tube.
By doing so, the structure can be simplified by reducing the number of component parts.

### {Advantageous Effects of Invention}

According to the present invention, an advantage is afforded that it is possible to recover a cell suspension containing a cell group from which unwanted components have been removed by efficiently washing the cell group.

### {Brief Description of Drawings}

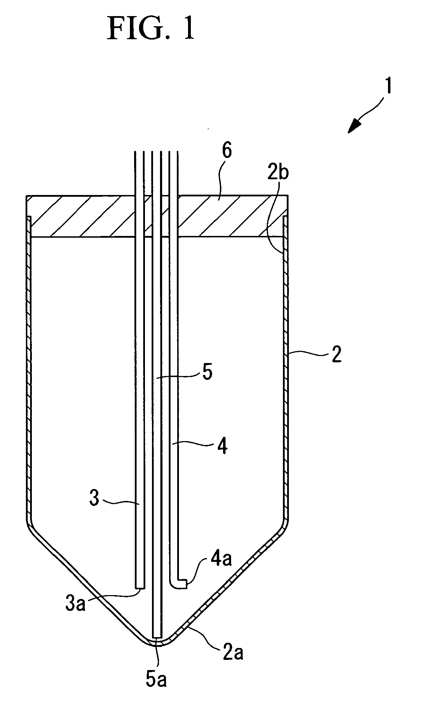

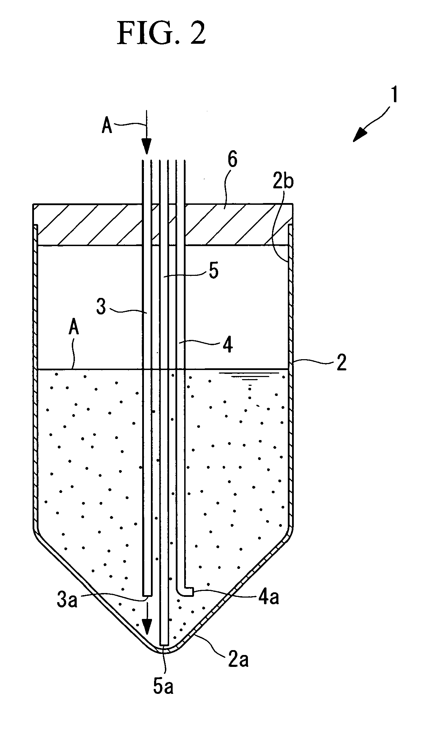

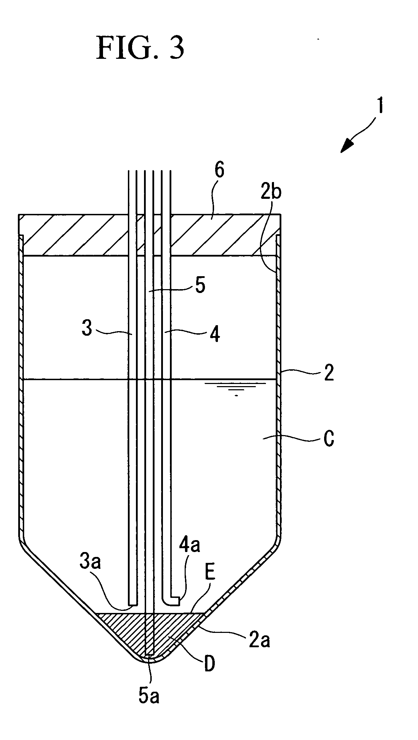

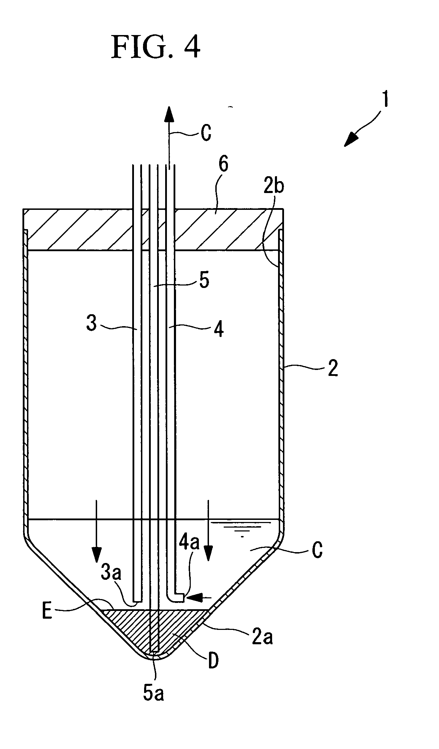

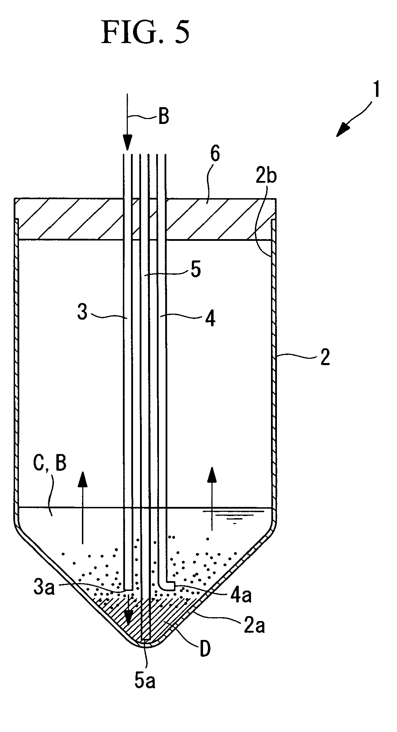

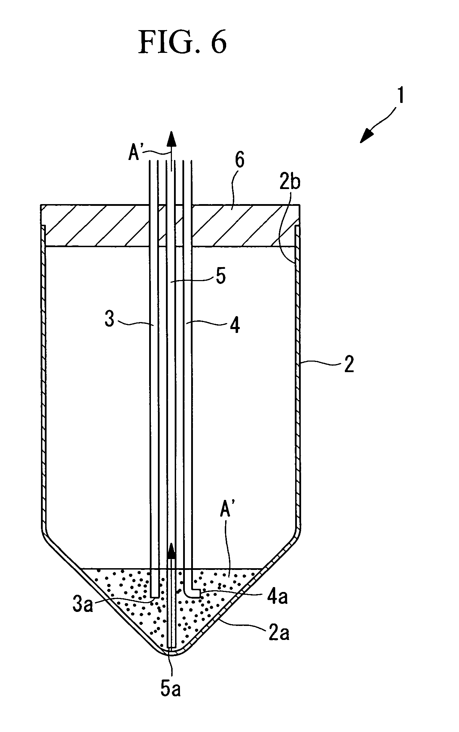

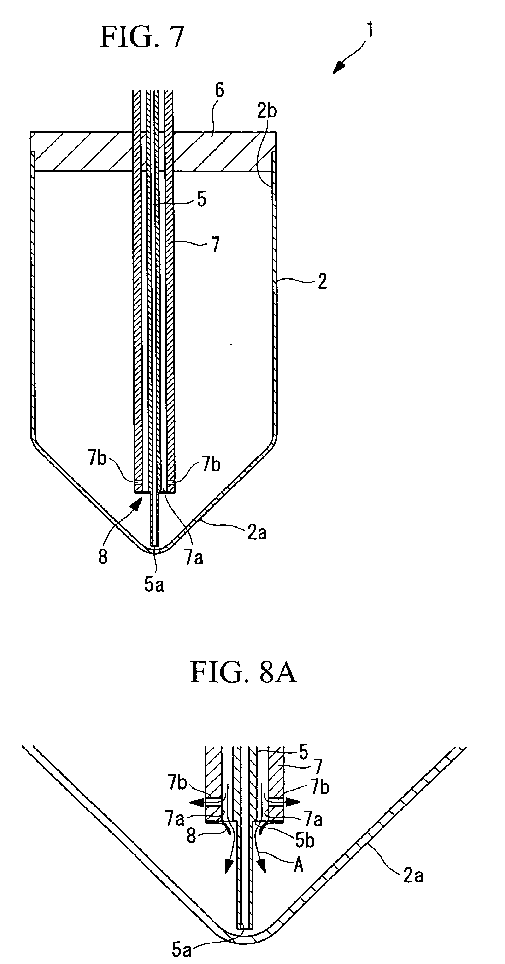

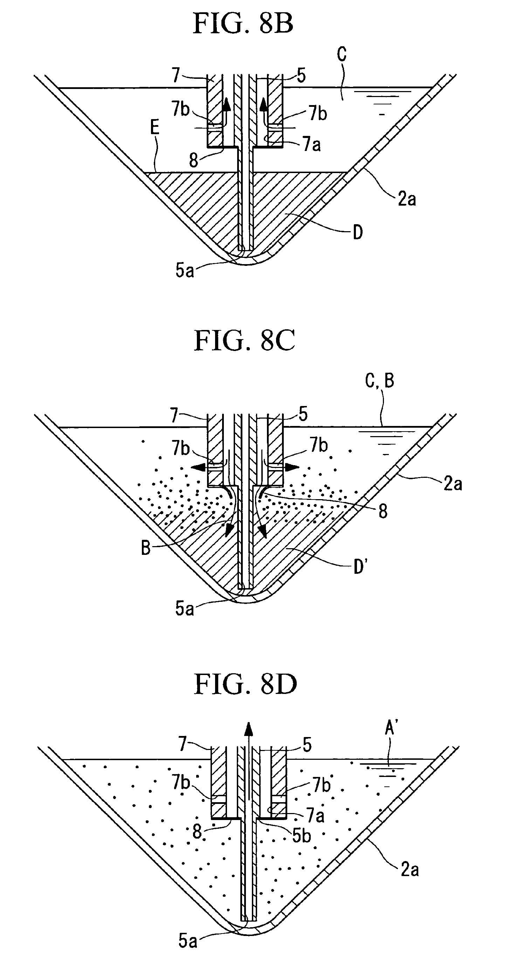

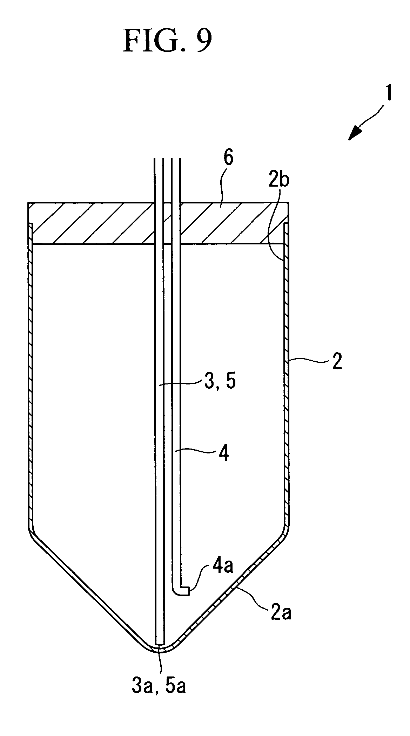

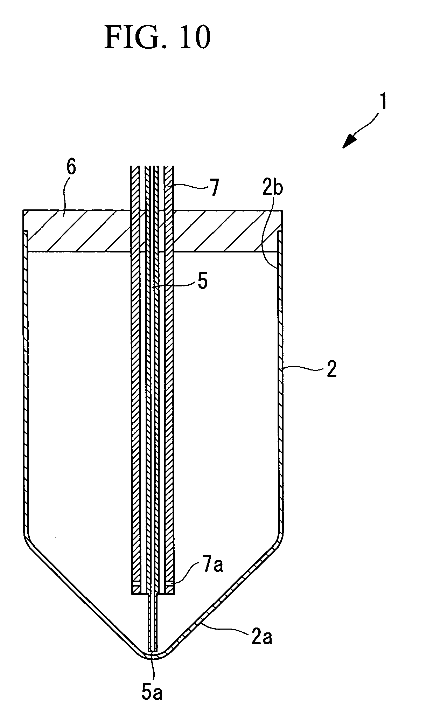

{Fig. 1} Fig. 1 is a longitudinal sectional view showing a centrifuge container according to an embodiment of the present invention.
{Fig. 2} Fig. 2 is a longitudinal sectional view showing a state where a cell suspension is accommodated in the centrifuge container shown in Fig. 1.
{Fig. 3} Fig. 3 is a longitudinal sectional view showing a state where the cell suspension accommodated in the centrifuge container shown in Fig. 1 is centrifuged.
{Fig. 4} Fig. 4 is a longitudinal sectional view for explaining a step of suctioning a supernatant from the cell suspension centrifuged in Fig. 3.
{Fig. 5} Fig. 5 is a longitudinal sectional view showing a step of supplying a washing fluid in the state shown in Fig. 4.
{Fig. 6} Fig. 6 is a longitudinal sectional view showing a step of suctioning and recovering the cell suspension resuspended in the centrifuge container shown in Fig. 1.
{Fig. 7} Fig. 7 is a longitudinal sectional view showing a first modification of the centrifuge container shown in Fig. 1.
{Fig. 8A} Fig. 8A is a partially-enlarged longitudinal sectional view showing a step of supplying a cell suspension, using the centrifuge container shown in Fig. 7.
{Fig. 8B} Fig. 8B is a partially-enlarged longitudinal sectional view showing a step of suctioning a supernatant, using the centrifuge container shown in Fig. 7.
{Fig. 8C} Fig. 8C is a partially-enlarged longitudinal sectional view showing a step of supplying a washing fluid, using the centrifuge container shown in Fig. 7.
{Fig. 8D} Fig. 8D is a partially-enlarged longitudinal sectional view showing a step of suctioning and recovering a cell group, using the centrifuge container shown in Fig. 7.
{Fig. 9} Fig. 9 is a longitudinal sectional view showing a second modification of the centrifuge container shown in Fig. 1.
{Fig. 10} Fig. 10 is a longitudinal sectional view showing a third modification of the centrifuge container shown in Fig. 1.

### {Description of Embodiments}

A centrifuge container 1 according to an embodiment of the present invention will be described below with reference to Figs. 1 to 6.
As shown in Figs. 1 to 6, the centrifuge container 1 according to this embodiment is provided with a cylindrical container main body 2 which is closed at one end forming a bottom portion 2a, a fluid introduction tube (suspension supply tube, washing-fluid discharge tube) 3 that introduces a cell suspension A (Fig. 2) and a washing fluid B (Fig. 5) to the container main body 2, a supernatant suction tube 4 that suctions a supernatant C (Fig. 3) centrifuged in the container main body 2, and a cell suction tube 5 that suctions a cell suspension A' (Fig. 6) that contains a cell group D (Fig. 3) centrifuged in the container main body 2.

The container main body 2 is formed into a substantially cylindrical shape and has an opening 2b at one end and the bottom portion 2a at the other end, which is closed. The bottom portion 2a is formed into a tapered inner-surface shape, whose diameter is gradually reduced toward the tip.
The container main body 2 is sealed when the opening 2b is closed by means of a lid member 6.

The fluid introduction tube 3, the supernatant suction tube 4, and the cell suction tube 5 are fixed in the lid member 6, the tubes passing through the center thereof and the tips of the tubes being disposed in the container main body 2.
As shown in Fig. 3, the fluid introduction tube 3 has a tip opening 3a disposed at the supernatant C side with respect to an interfacial surface E between the cell group D and the supernatant C, which are centrifuged in the container main body 2. The tip opening 3a opens toward the bottom portion 2a of the container main body 2 so as to discharge the supplied cell suspension A and washing fluid B toward the bottom portion 2a of the container main body 2.

The supernatant suction tube 4 has a tip opening 4a that opens radially outward at substantially the same position as the tip opening 3a of the fluid introduction tube 3. Thus, when the supernatant C in the container main body 2 is suctioned from the tip opening 4a, the supernatant C is radially suctioned from the tip opening 4a, disposed at the supernatant C side with respect to the interfacial surface E between the cell group D and the supernatant C.

The cell suction tube 5 has a tip opening 5a disposed at a position close to the bottom portion 2a of the container main body 2. Thus, all the cell suspension A' containing the cell group D, accommodated in the container main body 2, can be suctioned to the outside of the container main body 2.
Pumps (not shown) are connected to the fluid introduction tube 3, the supernatant suction tube 4, and the cell suction tube 5, which are connected to the container main body 2, such that the fluids A, B, C, and A' therein can be transferred.

The operation of the thus-configured centrifuge container 1 according to this embodiment will be described below.
In order to separately recover the desired cell group D from the cell suspension A by using the centrifuge container 1 of this embodiment, the centrifuge container 1 is set in a centrifuge separator (not shown), and, as shown in Fig. 2, the cell suspension A is externally supplied to the container main body 2 through the fluid introduction tube 3. In this state, the centrifuge separator is operated to rotate the centrifuge container 1.

The centrifuge container 1 is rotated with the bottom portion 2a of the container main body 2 being directed radially outward, thereby centrifugalizing the cell suspension A accommodated in the container main body 2. Thus, various components contained in the cell suspension A are centrifuged according to the differences in their specific gravities, as shown in Fig. 3.

Specifically, since the bottom portion 2a of the container main body 2 is formed into the tapered inner-surface shape, the cell group D, with a higher specific gravity, contained in the cell suspension A is collected at the tip of the bottom portion 2a along the tapered inner surface. Thus, the cell group D sinks to the tip of the bottom portion 2a of the container main body 2, thereby being separated from the rest of the supernatant C.

In this state, the centrifuge separator is stopped, and the centrifuge container 1 is disposed such that the bottom portion 2a of the container main body 2 is directed vertically downward. Then, as shown in Fig. 4, the supernatant C in the container main body 2 is suctioned through the supernatant suction tube 4. Since the tip opening 4a of the supernatant suction tube 4 is directed radially outward, the ambient supernatant C is suctioned radially inward. Thus, only the supernatant C can be suctioned while avoiding a disadvantage that the cell group D located in the bottom portion 2a of the container main body 2 is suctioned.

Next, as shown in Fig. 5, the washing fluid B is supplied to the container main body 2 through the fluid introduction tube 3. Since the tip opening 3a of the fluid introduction tube 3 is disposed at the supernatant C side with respect to the interfacial surface E between the supernatant C and the cell group D and is directed toward the bottom portion 2a of the container main body 2, the supplied washing fluid B is blown out toward the cell group D located in the bottom portion 2a of the container main body 2. Thus, even when the cell group D becomes solidified like a pellet, when the washing fluid B is blown out, the cell group D can be unsolidified and resuspended in the washing fluid B.

Specifically, during the centrifugation, some of unwanted components, such as proteolytic enzyme and fat, are incorporated into the cell group D, which is moved to the bottom portion 2a side of the container main body 2 according to the specific gravity, thereby forming a pellet that is solidified together with the cell group D. However, when the washing fluid B is blown out to unsolidify the pellet-like cell group D, the incorporated unwanted components can be released.

The centrifuge container 1 in which the cell suspension A resuspended in this way is accommodated is rotated again through the operation of the centrifuge separator, to execute centrifugation. Thus, the cell group D in which the fraction of unwanted components has been reduced can be separated. Then, after the supernatant C is removed with suction similarly to the above-described manner, a small amount of washing fluid B is supplied to the container main body 2 through the fluid introduction tube 3.

As a result, the washing fluid B is again blown out to the cell group D, thus forming a cell suspension A' in which the pellet-like cell group D is unsolidified and resuspended. In this state, as shown in Fig. 6, the cell suspension A' accommodated in the container main body 2 is suctioned through the cell suction tube 5. Since the tip opening 5a of the cell suction tube 5 is disposed close to the bottom portion 2a of the container main body 2, all of the cell suspension A' in the container main body 2 is recovered by suction.

In this way, according to the centrifuge container 1 of this embodiment, the washing fluid B can be discharged so as to be blown out to the cell group D, and, even when the cell group D separated through the centrifugation is solidified like a pellet, it is possible to unsolidify the cell group D to remove unwanted components and to easily perform suction for recovery.

Note that, in this embodiment, the supernatant suction tube 4 and the fluid introduction tube 3 are formed by separate piping; however, instead of this, as shown in Fig. 7, they may be structured by a common path 7 in which a valve 8 is provided for switching between the suction of the supernatant C and the discharge of the fluids A and B.
In the example shown in Fig. 7, a tip opening 7a of the common path 7, which is concentrically disposed radially outward from the cell suction tube 5, is disposed at the supernatant C side with respect to the interfacial surface E between the centrifuged supernatant C and cell group D, and the valve 8 formed of a valving element made of an elastic member is disposed on the tip opening 7a.

Tip openings 7b used for suctioning the supernatant C are radially formed so as to pass through the outer wall of the common path 7. Furthermore, the tip opening 7a, used for discharging the cell suspension A and the washing fluid B, is formed at the end of the common path 7 in the direction of the axis and is a gap between the common path 7 and the cell suction tube 5 facing in the direction toward the bottom portion 2a of the container main body 2.

As shown in Figs. 8A to 8D, the valve 8 is formed into a ring-plate shape, the outer-circumferential edge thereof is secured on the end surface of the common path 7, and the inner-circumferential edge thereof can be displaced in the axial direction. Furthermore, a step portion 5b against which the inner-circumferential edge of the valve 8 butts is provided on the outer-circumferential surface of the cell suction tube 5.

With this structure, when the cell suspension A or the washing fluid B is supplied through the common path 7, as shown in Figs. 8A and 8C, the inner-circumferential edge of the valve 8 is displaced toward the bottom portion 2a of the container main body 2 by the pressure of the supplied fluid, thus opening the valve 8 and discharging the cell suspension A or the washing fluid B toward the bottom portion 2a of the container main body 2. On the other hand, when the supernatant C is suctioned, as shown in Fig. 8B, the inner-circumferential edge of the valve 8 butts against the step portion 5b, provided on the outer-circumferential surface of the cell suction tube 5, thus closing the valve 8 and suctioning the supernatant C radially from the tip openings 7b. Then, as shown in Fig. 8D, the cell suspension A' containing the cell group D is recovered with suction through the cell suction tube 5, located at the center.
By doing so, the structure can be simplified by sharing the path.

Furthermore, as shown in Figs. 9 and 10, the fluid introduction tube 3 and the cell suction tube 5 may be integrally formed. Fig. 9 illustrates a case where the fluid introduction tube 3 and the cell suction tube 5 are arranged in parallel. Fig. 10 illustrates a case where the fluid introduction tube 3 and the cell suction tube 5 are concentrically arranged. The shared piping 7 can be selectively switched by a three-way valve or check valve (not shown) between the supply of the cell suspensions A and A' and the washing fluid B, and recovery of the cell suspension A' containing the cell group D by suction.

Since the cell suction tube 5 needs to recover almost all the cell suspension A' existing in the container main body 2 by suction, the tip opening 5a thereof needs to be disposed at a position close to the bottom portion 2a. When the washing fluid B is supplied through the common path 7, which is integrally formed with the cell suction tube 5, it is possible to discharge the washing fluid directly to the pellet-like cell group B, formed through centrifugation, thus unsolidifying the cell group B more effectively.

### {Reference Signs List}

A, A' cell suspension
B washing fluid
C supernatant
D cell group
1 centrifuge container
2 container main body
2a bottom portion
3 fluid introduction tube (washing-fluid discharge tube, suspension supply tube)
3a, 7a tip opening (second opening)
4 supernatant suction tube
4a, 7b tip opening (first opening)
5 cell suction tube
5a tip opening
5b step portion
6 lid member
7 common path
8 valve

## Claims

1. A centrifuge container comprising:
a cylindrical container main body that accommodates a cell suspension and is rotated with a bottom portion being directed radially outward;
a supernatant suction tube that has a first opening at a position in the depth direction of the container main body and that suctions a supernatant obtained by centrifuging the cell suspension, from the first opening, in the radial direction of the container main body; and
a washing-fluid discharge tube that has a second opening at a position in the depth direction of the container main body and that discharges a washing fluid from the second opening, in the axial direction toward the bottom portion of the container main body.

2. A centrifuge container according to claim 1, wherein the supernatant suction tube and the washing-fluid discharge tube are provided with a common path through which the supernatant and the washing fluid flow, and a valve that is provided at the end of the common path, that opens the second opening when the washing fluid is discharged, and that closes the second opening when the supernatant is suctioned.

3. A centrifuge container according to claim 1 or 2, further comprising a cell suction tube that has a third opening close to the bottom portion of the container main body and that suctions, from the third opening, a cell suspension that contains a cell group centrifuged in the bottom portion.

4. A centrifuge container according to one of claims 1 to 3, further comprising a suspension supply tube for supplying, to the container main body, a cell suspension that contains a cell group to be centrifuged.

5. A centrifuge container according to claim 4, wherein the suspension supply tube is formed of piping common to the washing-fluid discharge tube.
